(19) Europäisches Patentamt
European Patent Office
Office européen des brevets

(11) **EP 3 569 138 B1**

(12) **EUROPÄISCHE PATENTSCHRIFT**

(45) Veröffentlichungstag und Bekanntmachung des Hinweises auf die Patenterteilung:
**09.10.2024 Patentblatt 2024/41**

(21) Anmeldenummer: **19184329.1**

(22) Anmeldetag: **30.06.2017**

(51) Internationale Patentklassifikation (IPC):
*A61B 3/10* (2006.01)    *A61B 3/103* (2006.01)
*G02B 13/00* (2006.01)    *G02B 17/08* (2006.01)
*A61B 3/14* (2006.01)

(52) Gemeinsame Patentklassifikation (CPC):
**A61B 3/103; A61B 3/10; A61B 3/14;
G02B 19/0019; G02B 19/0061; G02B 19/0066;**
G02B 13/001

(54) **KOMPONENTE, COMPUTERPROGRAMM, SYSTEM UND KIT ZUR AUGENGLASBESTIMMUNG**

COMPONENT, COMPUTER PROGRAM, SYSTEM AND KIT FOR EYEGLASS DETERMINATION

COMPOSANT, PROGRAMME INFORMATIQUE, SYSTÈME ET ENSEMBLE DESTINÉS À LA DÉTERMINATION DES LUNETTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.06.2016 DE 102016112023**

(43) Veröffentlichungstag der Anmeldung:
**20.11.2019 Patentblatt 2019/47**

(62) Dokumentnummer(n) der früheren Anmeldung(en) nach Art. 76 EPÜ:
**17734722.6 / 3 478 153**

(73) Patentinhaber:
• **Carl Zeiss Vision International GmbH**
**73430 Aalen (DE)**
• **Carl Zeiss AG**
**73447 Oberkochen (DE)**

(72) Erfinder:
• **BREUNINGER, Tobias**
**89542 Herbrechtingen (DE)**

• **SCHÄFFEL, Frank**
**72076 Tübingen (DE)**
• **WAHL, Siegfried**
**73072 Donzdorf (DE)**
• **LINDIG, Karsten**
**99084 Erfurt (DE)**
• **OHLENDORF, Arne**
**72072 Tübingen (DE)**
• **CABEZA-GUILLÉN, Jesús-Miguel**
**73434 Aalen (DE)**

(74) Vertreter: **Kraus & Lederer PartGmbB**
**Thomas-Wimmer-Ring 15**
**80539 München (DE)**

(56) Entgegenhaltungen:
EP-A1- 2 924 977    WO-A1-2015/071779
WO-A1-2016/022215    CN-A- 103 653 654
CN-U- 204 260 054    US-A1- 2013 235 346

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft eine Komponente, ein System mit einer derartigen Komponente, sowie ein Kit mit einer derartigen Komponente und einem zugehörigen Computerprogramm zur Augenglasbestimmung, d.h. zur Messung eines Sehfehlers eines Auges.

Die Augenglasbestimmung ist nach Helmut Goersch, Wörterbuch der Optometrie, 2. Auflage, Verlag Bode GmbH & Co. KG, Pforzheim, 2001, Seite 26 die Gesamtheit aller Tätigkeiten zur Bestimmung der Dioptrienkombination (Sphäre, Zylinder, Prisma) für die Korrektion von Fehlsichtigkeiten und zur Bestimmung des Nahzusatzes bei Presbyopie. Die Augenglasbestimmung besteht aus einem monokularen Teil zur Bestimmung der Ametropie (Refraktionsbestimmung) und Presbyopie (Nahglasbestimmung) und einem binokularen Teil zur Bestimmung von Winkelfehlsichtigkeit. Durch die Augenglasbestimmung werden also Daten bereitgestellt, die das Sehvermögen der untersuchten Person charakterisieren.

Die Augenglasbestimmung ist ein wichtiger Bestandteil von Augenuntersuchungen. Bei derartigen Augenglasbestimmungen werden Sehfehler gemessen, welche dann zumindest teilweise mit Sehhilfen wie Brillen oder Kontaktlinsen ausgeglichen werden können.

Bei der Refraktionsbestimmung kann zwischen subjektiver Refraktionsbestimmung und objektiver Refraktionsbestimmung unterschieden werden. Verfahren zur subjektiven Refraktionsbestimmung beruhen dabei auf einer (subjektiven) Rückmeldung einer zu untersuchenden Person über ihre visuelle Wahrnehmung. Ein Beispiel ist dabei eine Messung auf Basis von Sehtafeln mit immer kleiner werdender Schrift oder immer kleiner werdenden Symbolen, wobei die zu untersuchende Person Rückmeldung gibt, welche Zeichen sie erkennen kann. Verfahren und Vorrichtungen zur objektiven Refraktionsbestimmung benötigen eine derartige Rückmeldung der zu untersuchenden Person über ihre visuelle Wahrnehmung hingegen nicht.

Die Photorefraktion ist ein derartiges objektives Refraktionsverfahren. Dieses Verfahren basiert auf einem Photo (d.h. einer Bildaufnahme) eines Lichtreflexes in der Pupille einer Person. Aus der Größe und Lage dieses Lichtreflexes kann der Refraktionszustand mit einer mathematischen Formel errechnet werden. Unter Refraktionszustand ist nach Helmut Goersch, Wörterbuch der Optometrie, 2. Auflage, Verlag Bode GmbH & Co. KG, Pforzheim, 2001, Seite 239 der Zustand des optischen Systems des Auges bezüglich seines Brechwertes im Verhältnis zu seiner Baulänge zu verstehen.

W. Wesemann und H. I. Wesemann erläutern in "Photorefraktion - Ein objektives Screening-Verfahren zur Refraktionsbestimmung", DOZ - Deutsche Optiker Zeitung, 11/92, Seiten 50 bis 54, dass man bei der Photorefraktion zwei physikalisch-optisch vollkommen unterschiedliche Verfahren unterscheidet, nämlich

1. Die von Howland und Howland (1974) entwickelte "isotrope" Photorefraktion und

2. die von Kaakinen (1979) vorgestellte "exzentrische" Photorefraktion.

Die Methode der "exzentrischen" Photorefraktion wird von anderen Autoren auch als "statische Photoskiaskopie" (Howland, 1980), "paraxiale Photorefraktion" (Tyler und Noreia, 1985) und "Photoretinoskopie" (Schaeffel et al., 1987; Howland, 1985) bezeichnet.

W. Wesemann und H. I. Wesemann führen weiter aus, dass das erste exzentrische Photorefraktometer, das von Kaakinen (1979) gebaut wurde, sowie auch die meisten nachfolgenden Geräte (Hay et al., 1983; Molteno et al" 1983; Bobier und Braddick 1985; Day und Noreia, 1986; Noreia et al" 1986) aus einer Spiegelreflexkamera mit langbrennweitigem Objektiv und einem Blitzgerät bestanden. Im Unterschied zu einer normalen Kamera ist der Blitz bei der exzentrischen Photorefraktion aber extrem nah bei dem Kameraobjektiv befestigt. Den Abstand der Blitzröhre (Lichtquelle) von der Eintrittspupille der Kamera ist in diesem Fall die Exzentrizität der Lichtquelle.

Wenn man mit einem so nahe bei der Kameralinse montierten Blitz ein emmetropes Auge photographiert, so erhält man auf dem Bild rot aufleuchtende Augen. Wenn man ein fehlsichtiges Auge photographiert, so erscheint zusätzlich ein halbmondförmiger Lichtreflex in der Pupille, aus dessen Lage und Größe sich die Fehlsichtigkeit ableiten lässt. Weitere Details entnimmt man den vorstehend genannten Publikationen. Das Verfahren der exzentrischen Photorefraktion ist beispielsweise auch unter der URL roorda.vision.berkeley.edu/photoref.htm, Stand 21. März 2016, der Berkeley Universität beschrieben.

Die DE 10 2007 031 923 A1 offenbart eine Vorrichtung zur exzentrischen Photorefraktion, die einen scannenden Lichtstrahl benutzt. Die Vorrichtung kann beispielsweise in einem Operationsmikroskop integriert sein. Damit eignet sich die dort beschriebene Vorrichtung insbesondere zum stationären Einsatz beispielsweise in Arztpraxen oder Krankenhäusern. Eine weitere Vorrichtung zur Augenuntersuchung, welche grundsätzlich - gegebenenfalls mit geringfügigen Modifikationen - zur objektiven Refraktionsbestimmung verwendet werden kann, ist in der US 2015/0002817 A1 beschrieben. Hier handelt es sich um einen relativ aufwendigen Aufbau, welcher ebenfalls vorwiegend zum Einsatz in Arztpraxen oder Kliniken bestimmt ist. Die DE 197 19 694 A1 offenbart eine weitere Vorrichtung zur objektiven Refraktionsbestimmung mittels exzentrischer Photorefraktion. Bei dieser Vorrichtung sind Lichtquellen in verschiedenen Abständen zu einer Kamera angeordnet und mit der Kamera in einem gemeinsamen Gehäuse fest verbaut.

Auch die EP 1 308 128 A2 offenbart eine Vorrichtung zur objektiven Refraktionsbestimmung mittels exzentrischer Photorefraktion. Bei dieser Vorrichtung können alle benötigten Komponenten in einem kompakten Gehäuse bereitgestellt sein, um somit eine mobile Vorrichtung bereitzustellen. Ein Abstand zwischen der Vorrichtung und einer zu untersuchenden Person kann mittels Ultraschallsensoren, mittels optischer Triangulation oder mittels einer Musterprojektion bestimmt werden, und dieser Abstand fließt dann in die Refraktionsbestimmung ein.

US 2013/235346 offenbart eine Augenglasbestimmung mittels Smartphone und Aufnahme von Augenbildern bei vorbestimmten Abständen.

Bei den oben beschriebenen Herangehensweisen werden speziell gefertigte Vorrichtungen zur objektiven Refraktionsbestimmung benutzt, die entsprechend teuer sind.

Zunehmend wurde in den letzten Jahren versucht, mobile, kostengünstige Möglichkeiten zur exzentrischen Photorefraktionsbestimmung bereitzustellen. Ein Ansatz hierzu ist, mobile Computervorrichtungen wie Smartphones oder Tablets zu verwenden. So wurde in einem Posterbeitrag Nr. D0055 auf der ARVO (Association for Research in Vision and Ophthalmology) Konferenz 2014, Programmnr. 436, "Photoscreening for Refractive Error and Strabismus With a Smartphone App", dessen Zusammenfassung unter http://www.arvo.org/webs/am2014/abstract/ sessions/114.pdf veröffentlicht ist, vorgeschlagen, eine exzentrische Photorefraktionsmessung mit einem Smartphone unter Verwendung eines integrierten Blitzes des Smartphones und einer entsprechenden Software (App) durchzuführen. Auch kommerzielle derartige Apps zur Durchführung einer exzentrischen Photorefraktion sind erhältlich. Nachweise finden sich z. B. unter http://www.gocheckkids.com/ und http://www.gocheckkids.com/downloads/ Poster CEI_poster APOS_Poster2014.pdf.

Bei einer derartigen Herangehensweise kann jedoch die Verwendung einer integrierten Blitzlichtquelle des Smartphones nachteilig sein, weil beispielsweise der Abstand zwischen integrierter Blitzlichtquelle und integrierter Kamera für die Photorefraktionsmessung ungünstig ist und/oder die integrierte Blitzlichtquelle in einem ungünstigen Spektralbereich emittiert. Zudem ist die integrierte Blitzlichtquelle meist nur als eine einzelne Lichtquelle ausgeführt.

Der Abstand zwischen der integrierten Blitzlichtquelle und der Kamera bestimmt zusammen mit anderen Parametern den Messbereich bei der exzentrischen Photorefraktion.

Der theoretische Messbereich von -D bis +D in Dioptrien, der mit einer Vorrichtung zur exzentrischen Photorefraktion erreicht werden kann, ist durch die folgende Formel beschrieben:

$$D = \frac{E}{2\,A\,DF\,R}$$

Dabei ist E die Exzentrizität der Lichtquelle, d.h. der Abstand zwischen einem effektiven Lichtaustrittsort der Lichtquelle und einer Eintrittspupille der Kamera. Der effektive Lichtaustrittsort der Lichtquelle ist der Ort, von dem ein Lichtstrahl austritt, um das Auge zu beleuchten. Liegen die Lichtquelle und die Kamera in einer Ebene senkrecht zu der optischen Achse der Kamera, entspricht die Exzentrizität dem Abstand zwischen dem Mittelpunkt der Lichtquelle und der Eintrittspupille der Kamera, wobei hier der Mittelpunkt der Eintrittspupille (üblicherweise auf der optischen Achse der Kamera) herangezogen wird. Bei einer herkömmlichen Blitzlichtquelle eines Smartphones stimmt der effektive Lichtaustrittsort dabei mit dem Mittelpunkt der Blitzlichtquelle überein. Liegen Lichtquelle und Kamera nicht in einer solchen Ebene, wird ein Versatz zwischen Lichtquelle und Kamera in Richtung senkrecht zur Ebene (vertikaler Versatz) im Rahmen dieser Anmeldung vernachlässigt. Bevorzugt ist dabei der vertikale Versatz deutlich kleiner als die Exzentrizität, z. B. weniger als 25% oder weniger als 10% der Exzentrizität. Unter der Exzentrizität der Beleuchtung ist dann also der Abstand zwischen der optischen Achse eines Kameraobjektivs der mobilen Computervorrichtung, die für die Aufnahme eines Photos zur Bestimmung der exzentrischen Photorefraktion bestimmt ist, und einem Mittelpunkt des effektiven Lichtaustrittsorts des von der Lichtquelle emittierten Lichts, das zum Beleuchten des Auges der Person während der Aufnahme des Photos bestimmt ist, zu verstehen. A ist der Abstand zwischen Auge und Kamera. DF steht für die sogenannte "Dark Fraction", d.h. den nicht ausgeleuchteten Anteil der Pupille, oder anders ausgedrückt der Anteil der Pupille. R steht für den Radius der Pupille, welcher z. B. ebenfalls dem aufgenommenen Bild entnehmbar ist (gegebenenfalls skaliert oder normiert basierend auf dem ermittelten Abstand).Zu bemerken ist, dass sich in der Praxis in Abhängigkeit von dem zu untersuchenden realen Auge Abweichungen von diesem theoretischen Messbereich ergeben können. Insbesondere kann der Messbereich in der Praxis asymmetrisch sein.

Wie aus der obigen Formel ersichtlich, ist der Messbereich für einen gegebenen Abstand zunächst umso größer, je größer die Exzentrizität ist. Allerdings sinkt bei großen Messbereichen die Genauigkeit der Bestimmung der Augenrefraktion für verglichen mit D kleinen Fehlsichtigkeiten, sodass es z. B. wünschenswert sein kann, eine kleinere Exzentrizität zur Beleuchtung zu verwenden. So können kleinere Fehlsichtigkeiten genauer gemessen werden. Bezüglich der Verwendung eines Smartphones zur objektiven Refraktionsbestimmung offenbart die DE 10 2015 100 147 A1 eine Vorrichtung mit einer Halterung zur Aufnahme eines Smartphones. Die Halterung kann am Kopf getragen werden. Zur objektiven Refraktionsmessung wird ein Bild des Auges mit einer Kamera aufgenommen, die auf derselben Seite wie eine Anzeige des Smartphones angeordnet ist. Diese Kamera wird üblicherweise als Frontkamera bezeichnet. Zur Beleuchtung des Auges können auf der Anzeige des Smartphones verschiedene Muster dargestellt werden. Zudem kann eine Lichtquelle im Inneren der Halterung vorgesehen sein, deren Licht über einen Strahlteiler zentral in den Strahlengang der Kamera eingespiegelt wird.

Die DE 2 232 410 A offenbart eine Vorrichtung zur Entfernungsmessung, bei der ein Prisma einen Strahlversatz erzeugt.

Ausgehend von der EP 1 308 128 A2 ist es eine dritte Aufgabe der vorliegenden Erfindung, Alternativen zu den in der EP 1 308 128 A2 genannten Möglichkeiten zur Abstandmessung bei der Photorefraktion bereitzustellen, welche ohne zusätzliche Komponenten oder nur mit einfacheren und kostengünstigeren zusätzlichen Mitteln als z.B. den Ultraschallsensoren oder der Vorrichtung zur Musterprojektion der EP 1 308 128 A2 implementierbar sind.

Hierzu werden in einem zweiten Erfindungsaspekt ein Verfahren nach Anspruch 1, ein Computerprogramm nach Anspruch 2 sowie eine mobile Computervorrichtung nach Anspruch 3 bereitgestellt.

In einem ersten, nicht beanspruchten Aspekt wird ein System bereitgestellt, umfassend:

> eine mobile Computervorrichtung, wobei die mobile Computervorrichtung ein Gehäuse und eine in dem Gehäuse eingebaute Kamera umfasst, gekennzeichnet durch mindestens eine Komponente aus der folgenden Gruppe:

> - eine Komponente mit einem Optikelement zum Anpassen der Exzentrizität einer integrierten Lichtquelle der mobilen Computervorrichtung und einem Befestigungselement zum reversibel lösbaren Verbinden der Komponente mit dem Gehäuse,
> - ein Hüllenelement, welches dimensioniert ist, die mobile Computervorrichtung ganz oder teilweise zu umhüllen, und welches mindestens eine Lichtquelle aufweist, wobei die mindestens eine Lichtquelle auf einer Außenseite des Hüllenelements angeordnet ist.

Zudem wird in dem ersten Aspekt eine Komponente bereitgestellt, umfassend:

> ein Optikelement zum Anpassen der Exzentrizität des Lichtaustritts einer integrierten Lichtquelle einer mobilen Computervorrichtung, und mindestens ein Befestigungselement zum Befestigen und Positionieren der Komponente an einem Gehäuse der mobilen Computervorrichtung.

Des Weiteren wird in dem ersten Aspekt eine Komponente umfassend ein Hüllenelement bereitgestellt, welches dimensioniert ist, die mobile Computervorrichtung ganz oder teilweise zu umhüllen, Das Hüllenelement weist mindestens eine Lichtquelle aufweist, wobei die mindestens eine Lichtquelle auf einer Außenseite des Hüllenelements angeordnet ist. Mit dem Optikelement kann also eine Exzentrizität der integrierten Lichtquelle der mobilen Computervorrichtung verändert werden, um eine für eine exzentrische Photorefraktionsmessung geeignete Exzentrizität (wie Eingangs erläutert) bereitzustellen. Alternativ kann mittels des Hüllenelements mindestens eine Lichtquelle mit einer geeigneten Exzentrizität auf einfache Weise bereitgestellt werden. Zudem kann mittels des Hüllenelements auf kompakte Weise eine Lichtquelle für die mobile Computervorrichtung bereitgestellt werden. Ein Optikelement ist also ein Element, das die Ausbreitung von Licht beeinflusst, z. B. durch Umlenkung des Lichts, um so die Exzentrizität anzupassen. Eine Komponente im Sinne dieser Anmeldung ist ein Bauteil, das zusammen mit der mobilen Computervorrichtung verwendbar ist und ein derartiges Optikelement oder ein Hüllenelement umfasst.

Ein Befestigungselement ist ein Element, mittels dem die Komponente an der mobilen Computervorrichtung befestigt und bevorzugt auch positioniert werden kann. Die Positionierung kann insbesondere dazu dienen, das Optikelement relativ zu der internen Lichtquelle zu positionieren, sodass das Optikelement Licht von der internen Lichtquelle empfängt. Das Befestigungselement kann beispielsweise ein Haftmittel wie eine Klebefolie, eine magnetische Befestigung oder auch eine mechanische Befestigung beispielsweise in Form eines Hüllenelements oder einer Klammer umfassen. Zur Positionierung kann beispielsweise eine Klebefolie oder eine magnetische Befestigung einen an der mobilen Computervorrichtung anzubringenden Teil aufweisen, der Markierungen zur Positionierung oder Aussparungen für Elemente der mobilen Computervorrichtung wie Kamera oder integrierte Lichtquelle aufweist, anhand derer die Positionierung erfolgt. Das Befestigungselement kann insbesondere zur reversibel lösbaren Befestigung der Komponente an der mobilen Computervorrichtung eingerichtet sein. Reversibel lösbar bedeutet hier, dass die Komponente zerstörungsfrei wieder von der mobilen Computervorrichtung entfernt werden kann und auch später wieder befestigt werden kann.

Ein Hüllenelement ist dabei ein Element, welches dimensioniert ist, die mobile Computervorrichtung zumindest teilweise zu umhüllen.

Durch ein derartiges Hüllenelement kann auf einfache Weise eine genaue Positionierung erfolgen, da es an die Abmessungen der mobilen Computervorrichtung angepasst ist. Diesbezüglich ist es dem Fachmann bekannt, dass für verschiedene Typen von mobilen Computervorrichtungen (z. B. Smartphones, Tablets) speziell angepasste Hüllenelemente erhältlich sind. Ein Hüllenelement ist dabei allgemein ein Element, in das die mobile Computervorrichtung aufnehmbar ist, so dass das Hüllenelement zusammen mit der mobilen Computervorrichtung eine Einheit bildet, wobei die Außenseite des Hüllenelements, an der die mindestens eine Lichtquelle angeordnet ist, eine sichtbare Außenseite dieser Einheit oder einen Teil (bei einem teilwesen Umhüllen) bildet. Dies unterschiedet sich von der Halterung der DE 10 2015 100 147 A1, bei der eine Lichtquelle im Inneren der Halterung bereitgestellt ist und nicht an einer Außenseite.

Unter der Außenseite des Hüllenelements ist dabei eine Seite zu verstehen, welche von der mobilen Computervorrichtung abgewandt ist, wenn das Hüllenelement die mobile Computervorrichtung ganz oder teilweise umhüllt, d.h. die Seite des Hüllenelements, die mit der mobilen Computervorrichtung in Kontakt steht, ist die Innenseite des Hüllenelements, und die Außenseite ist entsprechend die der Innenseite abgewandte Seite.

Wenn das Hüllenelement die mobile Computervorrichtung ganz umhüllt, ist die mobile Computervorrichtung von der Außenseite abgesehen von Aussparungen für Komponenten wie eine integrierte Kamera nicht sichtbar. Wenn das Hüllenelement die mobile Computervorrichtung teilweise umhüllt, sind Teile der mobilen Computervorrichtung sichtbar.

Ein solches Hüllenelement ist bevorzugt vergleichsweise dünn, d.h. weist einen geringen Abstand zwischen der Innenseite und der Außenseite auf, z. B. kleiner als 5 mm, oder kleiner als 3 mm. Das Hüllenelement weist dort, wo es die mobile Computervorrichtung ganz oder teilweise umhüllt, bevorzugt im Wesentlichen die gleiche Form wie die mobile Computervorrichtung auf, sodass sie die mobile Computervorrichtung im Wesentlichen formschlüssig umhüllt.

Eine mobile Computervorrichtung ist ein Gerät, bei welchem typischerweise ein oder mehrere Prozessoren, ein Speicher, eine Anzeige (Display) und gegebenenfalls weitere Bestandteile wie Schnittstellen und dergleichen in einem Gehäuse untergebracht sind. Die Anzeige kann dabei berührungsempfindlich sein (beispielsweise ein sogenannter Sensorbildschirm; engl.: touch screen), um somit auch Eingaben zu ermöglichen. Zudem umfassen mobile Computervorrichtungen, welche im Rahmen der vorliegenden Anmeldung verwendet werden, eine in dem Gehäuse eingebaute Kamera. Typische Beispiele für derartige mobile Computervorrichtungen sind Smartphones oder Tablet-PCs, oder gegebenenfalls auch LaptopComputer. Mobil ist die Computervorrichtung dann, wenn sie bei bestimmungsgemäßem Gebrauch von einer Person mit sich geführt werden kann.

Eine optische Achse entspricht üblicherweise einer geraden Verbindungslinie aller Krümmungsmittelpunkte von brechenden oder spiegelnden Flächen der abbildenden Optik. Lichtstrahlen auf der optischen Achse gehen ohne Ablenkung durch die abbildende Optik hindurch. Im Falle der Kamera der mobilen Computervorrichtung ist ein verwendetes Kameraobjektiv die abbildende Optik. Die optische Achse des Kameraobjektivs ist demnach in der Regel die gerade Linie, die mit der Symmetrieachse des Kameraobjektivs übereinstimmt und durch die Eintrittspupille des Kameraobjektivs mittig hindurchgeht.

Unter dem Emissionsspektrum der Beleuchtung ist die spektrale Verteilung des von der Lichtquelle (integrierten Lichtquelle oder mindestens einen Lichtquelle des Hüllenelements) emittierten und das Auge beleuchtenden Lichts zu verstehen. Dieses kann beispielsweise durch ein eingesetztes Leuchtmittel (z. B. LED) oder einen zusätzlichen, vorgesetzten Filter (z. B. einen Farbfilter oder einen dichroitischen Filter) beeinflusst werden. Unter Licht versteht man im Rahmen der vorliegenden Erfindung elektromagnetische Strahlung im Wellenlängenbereich zwischen 280 nm und 1200 nm. Es ist bekannt, dass Licht im ultravioletten Spektralbereich eine das Auge schädigende Wirkung haben kann.

Die spektrale Verteilung wird bevorzugt so gewählt, dass eine für die Messung ausreichende Menge Licht vom Fundus (Augenhintergrund) reflektiert wird (der sogenannte Rotreflex) und dass die Messung möglichst robuste Messwerte liefert. Die Bereitstellung einer hinreichenden Intensität an roter oder auch infraroter Strahlung ist demnach wünschenswert.

Durch einen hohen Anteil an kurzwelligem (z. B. blauem) Licht kann demgegenüber die zu vermessende Person geblendet werden, was zu einer Verkleinerung des Pupillendurchmessers führt, was wiederum die Messung erschweren kann. Aus diesem Grund wird der Intensitätsanteil ultravioletter oder blauer Strahlung in der Regel eher vergleichsweise gering gehalten, zumindest im Vergleich zum Intensitätsanteil roter oder infraroter Strahlung.

Bevorzugt umfasst das Befestigungselement eine Einstelleinrichtung zum Einstellen einer Position des Optikelements relativ zu der integrierten Lichtquelle der mobilen Computervorrichtung. Mittels einer derartigen Einstelleinrichtung kann das Optikelement genau positioniert werden, beispielsweise an verschiedene Positionen integrierter Lichtquellen bei verschiedenen mobilen Computervorrichtungen angepasst werden. Eine Einstelleinrichtung ist dabei eine Einrichtung, über die die Position des Optikelements veränderbar ist.

Das mindestens eine Optikelement kann dabei eingerichtet sein, von der internen Lichtquelle der mobilen Computervorrichtung, z. B. einer in das Gehäuse der mobilen Computervorrichtung integrierten Blitzlichtquelle oder einer in das Gehäuse der mobilen Computervorrichtung integrierten Infrarotlichtquelle, emittiertes und auf das Optikelement in einer Einfallsrichtung einfallendes Licht zu empfangen und das Beleuchtungslicht auf Basis des empfangenen Lichts versetzt oder parallel versetzt zu der Einfallsrichtung auszugeben.

Auf diese Weise kann durch Wahl des Versatzes zwischen einem Empfangsort auf einer Eintrittsfläche des Lichts in das Optikelement und einem effektiven Lichtaustrittsort auf einer Austrittsfläche des Lichts aus dem Optikelement eine gewünschte Exzentrizität der Beleuchtung zum Objektiv der verwendeten Kamera eingestellt werden.

Das mindestens eine Optikelement kann zu diesem Zweck ein Prisma umfassen. Ein Prisma ist ein geometrischer Körper, dessen Seitenkanten parallel und gleich lang sind und der ein Vieleck als Grundfläche hat. Ein Prisma entsteht durch Parallelverschiebung eines ebenen Vielecks entlang einer nicht in dieser Ebene liegenden Geraden im Raum und ist daher ein spezielles Polyeder. Besonders günstig ist es, wenn das Prisma als planparallele Platte ausgebildet ist.

Das Prisma kann z. B. aus einem Glas- oder Kunststoffmaterial gefertigt sein. Ein Kunststoffprisma kann z. B. kostengünstig durch ein Spritzgussverfahren hergestellt werden.

Mittels eines derartigen Prismas kann auf kostengünstige Weise Licht einer eingebauten Lichtquelle der Computervorrichtung eine Beleuchtung für die Durchführung einer exzentrischen Photorefraktion bereitstellend auf einen geeigneten Abstand zur Kamera umgelenkt werden.

Statt eines Prismas kann auch eine entsprechend eingerichtete optische Folie verwendet werden, welche im Wesentlichen die gleiche Funktion hat, d.h. eine Folie, bei der Licht von der Lichtquelle an Grenzflächen mit Brechungsindexsprung innerhalb der Folie oder zwischen der Folie und der Umgebung der Folie durch Brechung oder Reflexion abgelenkt wird, um einen Versatz und somit eine gewünschte Exzentrizität einzustellen.

Das mindestens eine Optikelement weist bei einem bevorzugten Ausführungsbeispiel eine Lichteintrittsfläche, welche eingerichtet ist, von der integrierten Lichtquelle der mobilen Computervorrichtung, emittiertes Licht zu empfangen und eine Lichtaustrittsfläche, welche eingerichtet ist, das empfangene Licht auszugeben, auf. Das Optikelement ist ferner derart ausgebildet, dass das empfangene Licht innerhalb eines Toleranzbereichs von weniger als 1 mm oder weniger als 0,5 mm stets an derselben Stelle der Lichtaustrittsfläche ausgegeben wird, unabhängig davon, an welcher Stelle das von der integrierten Lichtquelle der mobilen Computervorrichtung emittierte Licht durch die Lichteintrittsfläche empfangen worden ist.

Hierdurch kann unabhängig von einer genauen Position der eingebauten Lichtquelle der mobilen Computervorrichtung, beispielsweise eines Blitzes, eine effektive Position und Achse der Beleuchtung relativ zu einer Kamera der mobilen Computervorrichtung und somit die Exzentrizität festgelegt werden.

Eine solche Optikkomponente der beschriebenen Art umfasst dabei bevorzugt eine Vielzahl der Lichteintrittsfläche zugeordneter erster brechenden oder reflektierenden Flächen, beispielsweise Prismenflächen, die derart geneigt sind, dass sie über die Lichteintrittsfläche empfangenes Licht in eine gleiche Richtung lenken, z. B um etwa 90° ablenken. Eine Prismenfläche ist dabei eine Fläche eines Prismas, an der ein Brechungsindexsprung (zwischen dem Prisma und der Umgebung, z. B. Luft oder einem anderen Material) stattfindet und somit eine Reflexion der Brechung erfolgt, durch die das Licht abgelenkt wird. Strukturgrößen derartiger erster Flächen können unterhalb von 1 mm liegen, aber auch größer sein. Zudem umfasst die Optikkomponente in diesem Fall eine zweite brechende oder reflektierende Fläche, um das von den ersten brechenden oder reflektierenden Flächen abgelenkte Licht zu der Lichtaustrittsfläche zu lenken, z. B. nochmals um etwa 90° abzulenken. Im Falle von Prismenflächen kann die Anordnung erster Flächen als Mikro-Prismen-Array bezeichnet werden.

Statt der oben beschriebenen Strukturen mit den brechenden oder reflektierenden ersten und zweiten Flächen können auch diffraktive (d.h. beugende) Strukturen und Elemente mit solchen diffraktiven Strukturen (kurz als diffraktive Elemente bezeichnet) zur Ablenkung des Lichts verwendet werden. Diffraktive Strukturen weisen typischerweise so kleine Strukturgrößen, z. B. im Bereich der Lichtwellenlänge des Lichts, dass Beugungseffekte auftreten. Durch geeignete Ausgestaltung der diffraktiven Strukturen kann Licht durch Beugung unter einem durch die Ausgestaltung der Strukturen bestimmten Winkel abgelenkt werden.

Diffraktive Strukturen können bevorzugt in Form eines Hologramms, z. B. in Form eines holografischen Volumengitters, bereitgestellt werden. Zur Herstellung eines derartigen holografischen Volumengitters wird beispielsweise wie für sich genommen bekannt ein lichtempfindliches Material mit zumindest zwei interferierenden kohärenten Lichtwellen beleuchtet, wobei eine Lichtwelle aus einer Richtung entsprechend der Position der Lichtquelle der mobilen Computervorrichtung kommt und die andere Lichtwelle aus einer Richtung entsprechend einer gewünschten Ablenkrichtung, in die das Licht von der Lichtquelle abgelenkt werden soll, kommt. Anschließend wird das lichtempfindliche Material entwickelt.

Durch eine solche, günstig herzustellende Optikkomponente lässt sich Licht, welches in dem Bereich, z. B. von verschiedenen möglichen Positionen einer eingebauten Beleuchtung oder mobilen Computervorrichtung, empfangen wird, auf einfache Weise weiterleiten.

Die mindestens eine Lichtquelle des Hüllenelements kann beispielsweise eine oder mehrere Leuchtdioden (LEDs), eine oder mehrere organische Leuchtdioden (OLEDs) oder ein oder mehrere Quantenpunktlichtquellenelemente umfassen.

Durch die Bereitstellung von einer oder mehreren Lichtquellen in der Komponente kann beispielsweise ein gewünschter Spektralbereich der Beleuchtung und/oder ein gewünschter effektiver Lichtaustrittsort der Beleuchtung und somit die Exzentrizität auf einfache Weise festgelegt werden. Auch eine Form der Beleuchtung oder eine Beleuchtung aus verschiedenen Richtungen kann auf einfache Weise realisiert werden.

Die Lichtquellen können beispielsweise durch einen in der Komponente angeordneten Akkumulator versorgt werden. Die Lichtquellen können auch über eine Stromversorgung einer mobilen Computervorrichtung versorgt werden, beispielsweise über eine entsprechende drahtgebundene Kopplung mit einer Schnittstelle der mobilen Computervorrichtung (beispielsweise einer USB-Schnittstelle).

Beispielsweise kann die mindestens eine Lichtquelle des Hüllenelements eine Infrarotlichtquelle sein.

Dies hat den Vorteil, dass sich die Pupille eines zu untersuchenden Auges durch die Beleuchtung nicht verengt und eine Fundusreflektivität des Auges, d.h. einer Reflexion des Lichts am Augenfundus, weniger stark individuellen Schwankungen unterliegt.

Insbesondere kann die mindestens eine Lichtquelle des Hüllenelements eine Vielzahl keilförmig angeordneter Lichtquellen umfassen. Durch eine Beleuchtung mit derartig keilförmig angeordneten Lichtquellen kann eine Reflexform in Abhängigkeit von der Refraktion des Auges linearisiert werden, da sich gezeigt hat, dass die Intensitätsverteilung des Rotreflexes in Abhängigkeit von einer Fehlsichtigkeit der zu untersuchenden Person sich bei einer derartigen keilförmigen Beleuchtung einen linearen Verlauf aufweist. Dies kann die Auswertung von aufgenommenen Bildern des Auges zur Refraktionsbestimmung erleichtern, da in diesem Fall einfach die Steigung dieses linearen Verlaufs ausgewertet werden kann. Diese Herangehensweise ist in der eingangs genannten URL der Berkeley Universität unter Verweis auf Schaeffel, F., Farkas, L. & Howland, H.C. (1987) Infrared photoretinoscope. Appl. Opt. 26, 1505-1509 näher erläutert.

Die mindestens eine Lichtquelle des Hüllenelements kann mehrere in verschiedenen Abständen von der Kamera der mobilen Computervorrichtung anzuordnende Lichtquellen umfassen. So kann durch entsprechende Aktivierung von verschiedenen Lichtquellen eine gewünschte Exzentrizität der Beleuchtung eingestellt werden, was wie eingangs erläutert zur Einstellung eines gewünschten Messbereichs bei einem bestimmten Abstand zwischen Auge und Kamera verwendet werden kann.

Die Komponente kann ein Filter zum Filtern des Beleuchtungslichts und/oder ein Filter zum Filtern von für eine integrierte Kamera der mobilen Computervorrichtung bestimmten Lichts umfassen.

Hierdurch kann ein für eine Refraktionsmessung gewünschtes Spektrum durch entsprechende Wahl des Filters festgelegt werden, insbesondere ein Spektrum zumindest vorwiegend in roten und/oder infraroten Bereich wie weiter oben erläutert.

Das Hüllenelement kann eine Öffnung für eine Kamera der mobilen Computervorrichtung umfassen, wobei die mindestens eine Lichtquelle, derart benachbart zu der Öffnung angeordnet sein kann, dass eine gewünschte Exzentrizität, z. B. zwischen 5 mm und 20 mm, gegeben ist, z. B. zum Erreichen eines gewünschten Messbereichs z. B. zwischen -2 und 2 Dioptrien, oder zwischen -5 und 5 Dioptrien (wobei dann kleinere Fehlsichtigkeiten gegebenenfalls nicht messbar sind) bei einem bestimmten Abstand (z. B. im Bereich 40-60 cm) wie eingangs erläutert, wobei der Messbereich insbesondere ein einer zu untersuchenden Person angepasst sein kann. Auf diese Weise ist eine genaue Positionierung der Lichtquelle relativ zu der Kamera möglich, indem die Öffnung mit der Kamera zur Deckung gebracht wird. In anderen Worten wird die Öffnung so auf der mobilen Computervorrichtung angeordnet, dass die Kamera durch die Öffnung hindurch Bilder aufnehmen kann.

Zudem wird in dem ersten, nicht beanspruchten Aspekt ein Verfahren zur Augenglasbestimmung bereitgestellt, umfassend:

Beleuchten eines Auges einer Person mit Licht von einer integrierten Lichtquelle (22) einer mobilen Computervorrichtung,

Aufnehmen eines Bildes des Auges der Person mit einer Kamera (23) der mobilen Computervorrichtung, und

Durchführen einer exzentrischen Photorefraktionsbestimmung auf Basis des aufgenommenen Bildes, gekennzeichnet durch

Anpassen einer Exzentrizität des Lichtes auf eine Exzentrizität für die exzentrische Photorefraktionsbestimmung.

Durch das Anpassen der Exzentrizität, z. B. mittels des oben beschriebenen Optikelements, kann eine geeignete Exzentrizität für die exzentrische Photorefraktionsbestimmung eingestellt werden, wie oben bereits beschrieben.

Durch die exzentrische Photorefraktionsbestimmung werden dabei wie eingangs allgemein für die Augenglasbestimmung erläutert Daten bereitgestellt, die das Sehvermögen der Person kennzeichnen, beispielsweise als sphärozylindrische Refraktion (Sphäre, Zylinder, Achslage wie in der DIN EN ISO 13666:2012 definiert). Auf Basis dieser Daten kann z. B. ein Augenarzt dann eine Diagnose stellen, z. B. ob die Person weitsichtig oder kurzsichtig ist, und eine entsprechende Behandlung, z. B. das Tragen einer Brille, verordnen. Diese Diagnose ist nicht Gegenstand der vorliegenden Anmeldung, die beanspruchten Verfahren liefern lediglich die Daten, die dann zur Diagnose dienen können.

Die Exzentrizität kann unabhängig von einer Position der integrierten Lichtquelle innerhalb eines Toleranzbereichs auf die gewünschte Exzentrizität angepasst werden, wie bereits oben für manche Optikelemente beschrieben. Auf diese Weise kann das Verfahren trotz variierender Positionen der integrierten Lichtquelle (z. B. bei verschiedenen Typen mobiler Computervorrichtungen) auf die gleiche Exzentrizität eingestellt werden.

In dem zweiten Aspekt wird ein Verfahren durchgeführt, das im Anspruch 1 definiert ist.

Das Aufnehmen des Bildes kann dabei mehrmals mit verschiedenen Beleuchtungsrichtungen erfolgen. Hierdurch werden mehrere Meridiane vermessen, d.h. das Auge wird entlang verschiedener Richtungen vermessen. Hierdurch können Parameter wie Sphäre, Zylinder und Achse bestimmt werden.

Durch ein derartiges Computerprogramm kann eine mobile Computervorrichtung wie ein Smartphone zur exzentrischen Photorefraktion verwendet werden. Durch die Bestimmung des Abstands kann ein gewünschter Abstand festgelegt werden und somit bei gegebener Exzentrizität ein gewünschter Messbereich festgelegt werden, entsprechend dem eingangs erläuterten Zusammenhang zwischen Messbereich, Abstand und Exzentrizität.

Wie bereits erwähnt, wird bei Ausführungsbeispielen die exzentrische Photorefraktion verwendet, um die Augenglasbestimmung durchzuführen. Auch wenn hier die Augenglasbestimmung eines Auges diskutiert wird, ist hier "eines" lediglich als unbestimmter Artikel zu sehen, und es können selbstverständlich beide Augen einer Person untersucht werden. Dabei wird durch eine mittels einer der oben beschriebenen Varianten einer Komponente erzeugbaren exzentrischen Beleuchtung ein sogenannter Rotreflex, d.h. ein vom Fundus des Auges reflektiertes Licht, erzeugt. Dieser Rotreflex ist auf dem aufgenommenen Bild oder den aufgenommenen Bildern sichtbar. Liegt bei der Person eine Ametropie (Fehlsichtigkeit, d.h. Hyperopie, Myopie oder Astigmatismus) vor, so führt dies zu einer inhomogenen Ausleuchtung der Pupille. Bei einem emmetropen (normalsichtigem) Auge erscheint die Pupille dunkel. Mittels Bildanalyseverfahren kann die Lichtverteilung ausgewertet werden und auf Basis der Lichtverteilung die Fehlsichtigkeit der Person ermittelt werden. Hierzu werden mathematische Formeln, wie in der Literatur beschrieben und später kurz erläutert, herangezogen werden. Zu beachten ist, dass bei ungünstiger Exzentrizität der Beleuchtung auch bei einer vorliegenden Ametropie (Fehlsichtigkeit) die Pupille dunkel erscheinen kann. Mit den beschriebenen optischen Komponenten ist jedoch eine gewünschte Exzentrizität einstellbar.

Erfindungsgemäß umfasst das Bestimmen des Abstands dabei einen oder mehrere der folgenden Schritte:

- Bestimmen des Abstands auf Basis einer Autofokuseinstellung einer Kamera der mobilen Computervorrichtung,
- Bestimmen des Abstands auf Basis einer Aufnahme der mobilen Computervorrichtung über einen Spiegel,
- Setzen eines Autofokusses auf einen vorgegebenen Abstandswert, und Ausgeben einer Meldung, wenn ein Abstand des Kopfes dem vorgegebenen Abstandswert entspricht.

Der vorgegebene Abstandswert ist dabei ein Abstandswert, der für eine exzentrische Photorefraktionsbestimmung auf Basis der Eingangs erläuterten Zusammenhänge zwischen Messbereich, Exzentrizität und Abstand gewünscht bzw. benötigt ist.

Es gibt also eine Vielzahl von einfachen Möglichkeiten, den Abstand zu bestimmen. Diese erfolgen mit Komponenten, die entweder ohnehin vorhanden sind (wie der Autofokus der Kamera) oder mit kostengünstigen Komponenten wie einem Gegenstand bekannter Abmessungen oder einem Spiegel.

Zudem wird ein Computerprogramm mit einem Programmcode bereitgestellt, der, wenn er auf einem Prozessor ausgeführt wird, das obige Verfahren durchführt.

Ein derartiges Computerprogramm kann zum Beispiel zum Herunterladen aus einem Netzwerk auf die mobile Computervorrichtung bereitgestellt werden. Derartige Computerprogramme für mobile Computervorrichtungen werden auch als Apps bezeichnet. Bei dem Aufnehmen kann dabei ein Beleuchten des Auges unter Verwendung einer oder mehrerer der oben beschriebenen Komponenten erfolgen. Das Computerprogramm kann auch auf einem computerlesbaren Medium gespeichert sein.

Zudem wird eine mobile Computervorrichtung mit einem Prozessor und einem Datenträger (z. B. einem Speicher), auf dem ein derartiges Computerprogramm gespeichert ist, bereitgestellt.

Zudem wird ein Kit bereitgestellt, welches den ersten und zweiten Aspekt verknüpft.

Mit einem derartigen Kit kann eine kostengünstige Möglichkeit bereitgestellt werden, eine mobile Computervorrichtung wie ein Smartphone oder ein Tablet zur Refraktionsmessung zu verwenden, indem z. B. die Komponente an der mobilen Computervorrichtung angebracht wird und das Computerprogramm auf der mobilen Computervorrichtung ausgeführt wird.

Die vorliegende Erfindung wird nachfolgend anhand von verschiedenen Ausführungsbeispielen noch näher erläutert. Es zeigen:

Fig. 1 eine schematische Darstellung eines Systems gemäß einem Ausführungsbeispiel,

Fig. 2 eine perspektivische Teilansicht eines Systems gemäß einem Ausführungsbeispiel,

Fig. 3 eine Teilquerschnittsansicht eines Systems gemäß einem Ausführungsbeispiel,

Fig. 4 eine Darstellung eines Systems gemäß einem weiteren Ausführungsbeispiel,

Fig. 5 eine Darstellung einer Lichtquellenanordnung, welche in Komponenten gemäß manchen Ausführungsbeispielen verwendbar ist,

Fig. 6 ein System gemäß einem weiteren Ausführungsbeispiel,

Fig. 7 ein Flussdiagramm zur Veranschaulichung eines Verfahrens gemäß einem Ausführungsbeispiel,

Fig. 8A-8D Diagramme zur Veranschaulichung der Auswertung einer Bildaufnahme bei manchen Ausführungsbeispielen,

Fig. 9 eine Darstellung zur Veranschaulichung einer Ausrichtung eines Smartphones gemäß einem Ausführungsbeispiel, und

Fig. 10 ein System gemäß einem weiteren Ausführungsbeispiel.

Im Folgenden diskutierte Ausführungsbeispiele beziehen sich auf eine Komponente für eine mobile Computervorrichtung, insbesondere zum Anbringen an einem Gehäuse der mobilen Computervorrichtung. In der folgenden Beschreibung werden Smartphones als Beispiele für derartige mobile Computervorrichtungen verwendet. Erfindungsgemäße Systeme, welche im Folgenden erläutert werden, umfassen ein Smartphone und eine erfindungsgemäße Komponente, welche zum Beleuchten eines Auges einer zu untersuchenden Person dient. Als Alternative zu derartigen Smartphones können auch andere mobile Computervorrichtungen wie Tablet-PCs oder Laptopcomputer verwendet werden.

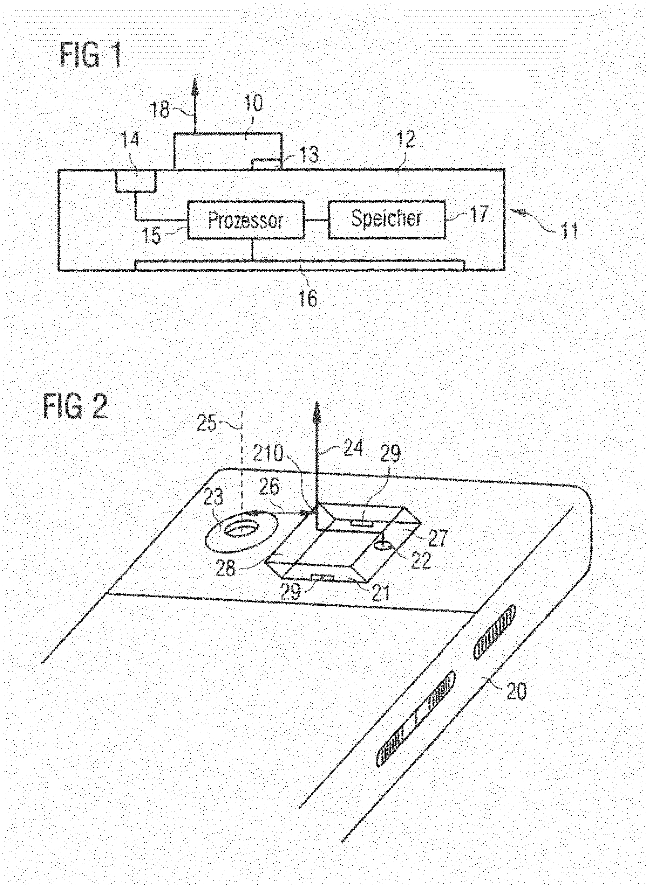

Die Fig. 1 zeigt eine schematische Darstellung eines Systems gemäß einem Ausführungsbeispiel. Das System der Fig. 1 umfasst eine mobile Computervorrichtung 11 und eine an einem Gehäuse 12 der mobilen Computervorrichtung 11 angebrachte Komponente 10. Die mobile Computervorrichtung 11 weist einen in dem Gehäuse 12 angeordneten Prozessor 15 sowie einen zugeordneten Speicher 17 auf. In dem Speicher 17 können insbesondere Programme abgelegt sein, welche, wenn sie auf dem Prozessor 15 laufen, bewirken, dass ein Verfahren zur Augenglasbestimmung eines Auges einer zu untersuchenden Person mittels exzentrischer Photorefraktion durchgeführt wird.

Die mobile Computervorrichtung 11 umfasst weiterhin eine in dem Gehäuse 12 eingebaute Kamera 14, über die ein oder mehrere Bilder einer zu untersuchenden Person, insbesondere eines oder beider Augen der Person, aufgenommen werden können. Des Weiteren umfasst die mobile Computervorrichtung 11 eine Anzeige 16, über die beispielsweise Ergebnisse der Refraktionsmessung ausgegeben werden. Die Anzeige 16 kann auch als sogenannter Sensorbildschirm ausgestaltet sein und Eingaben eines Benutzers ermöglichen.

Die dargestellten Bestandteile der mobilen Computervorrichtung 11 sind nicht als einschränkend auszulegen, und es können weitere (konventionelle) Bauteile von mobilen Computervorrichtungen wie Smartphones, Tablet-PCs oder Laptops bereitgestellt sein, beispielsweise Schnittstellen, Tastaturen, Kommunikationseinrichtungen zur Kommunikation mit einem Netzwerk (drahtlos oder drahtgebunden), Massenspeicher wie Festplatten und dergleichen.

Die Komponente 10 ist über ein Befestigungselement 13 an dem Gehäuse 12 der mobilen Computervorrichtung 11 angebracht.

Die Komponente 10 ist eingerichtet, Beleuchtungslicht zum Beleuchten des Kopfes der Person, insbesondere des Auges, auszugeben, wie durch einen Pfeil 18 angedeutet. Wie im Folgenden anhand verschiedener konkreter Ausführungsbeispiele näher erläutert werden wird, kann die Komponente 10 beispielsweise Licht von einer eingebauten Lichtquelle der mobilen Computervorrichtung 11 benutzen und diese geeignet umlenken und/oder filtern, um das Beleuchtungslicht 18 zu erzeugen. Derartige eingebaute Lichtquellen können zum Beispiel Blitzlichtquellen sein. Bei anderen Varianten kann die Komponente 10 ein Hüllenelement mit einer oder mehreren Lichtquellen umfassen, um das Beleuchtungslicht 18 zu erzeugen.

Der Prozessor 15 kann dabei bei manchen Ausführungsbeispielen das Beleuchtungslicht 18 steuern, sodass die Beleuchtung abgestimmt zu einer Bildaufnahme mit der Kamera 14 erfolgt. Bei anderen Ausführungsbeispielen kann die Komponente 10 beispielsweise einen Schalter oder dergleichen umfassen, mit dem die Beleuchtung ein- und ausgeschaltet werden kann. Mittels des Prozessors 15 werden schließlich aufgenommene Bilder zur Augenglasbestimmung ausgewertet.

Nunmehr werden unter Bezugnahme auf die Figuren 2-6 verschiedene Implementierungsmöglichkeiten für die Komponente 10 erläutert. Um Wiederholungen zu vermeiden, tragen in den Figuren 2-6 gleiche oder einander entsprechenden Elemente die gleichen Bezugszeichen und werden nicht wiederholt erläutert. In den Figuren 2-6 dient dabei ein Smartphone 20 als Beispiel für eine mobile Computervorrichtung. Die dargestellten Varianten der Komponente 10 lassen sich aber auch zusammen mit anderen mobilen Computervorrichtungen, beispielsweise Tablet-Computern, entsprechend verwenden.

Die Figur 2 zeigt eine perspektivische Teilansicht eines Systems umfassend ein Smartphone 20 und eine Komponente 21 gemäß einem zweiten Ausführungsbeispiel. Das Smartphone 20 weist rückseitig eine in das Gehäuse integrierte Lichtquelle 22 und eine in das Gehäuse integrierte Kamera 23 auf, die in einem Abstand von beispielsweise 10 mm zueinander angeordnet sind. Dieses Smartphone 20 ist zur Durchführung einer exzentrischen Photorefraktion unter Verwendung der integrierten Lichtquelle 22 und der integrierten Kamera 23 aufgrund der inhärenten Exzentrizität von 10 mm nicht für alle Abstände und Messbereiche geeignet. Wird das Smartphone beispielsweise in einem Abstand von 60 cm von einem zu untersuchenden Auge gehalten, ergibt sich ein Messbereich von etwa -5 bis +5 Dioptrien, wobei kleinere Fehlsichtigkeiten im Bereich von etwa -1 bis 1 Dioptrien nicht messbar sind. Auf der anderen Seite sind größere Abstände zumindest dann, wenn die zu untersuchende Person das Smartphone 20 selbst hält, auf Grund der beschränkten Armlänge schwierig zu erreichen. Daher ist bei dem System der Fig. 2 auf der Rückseite des Smartphones 20 (d.h. einer von der Anzeige des Smartphones abgewandten Seite) eine erfindungsgemäße Komponente 21 angebracht. Die Komponente 21 ist dabei so positioniert und ausgestaltet, dass sie die Exzentrizität der Beleuchtung auf einen gewünschten Wert ändert.

Die Komponente weist in dem in Fig. 2 gezeigten Ausführungsbeispiel hierzu ein Prisma 21, genauer gesagt eine planparallele Platte mit stirnseitig parallel zueinander angeordneten Reflexionsflächen 27, 28, als Optikelement auf, welches bei dem dargestellten Ausführungsbeispiel durch Haftstreifen 29 an dem Gehäuse des Smartphones 20 angebracht ist. Das Prisma 21 lenkt Licht, welches von einer internen Lichtquelle 22 des Smartphones 20 ausgesendet wird, wie in Fig. 2 dargestellt um, indem das von der Lichtquelle 22 emittierte Licht wie dargestellt zunächst an der Reflexionsfläche 27 und dann an der Reflexionsfläche 28 reflektiert wird, um Beleuchtungslicht 24 in einem gewünschten Abstand zu einer Kamera 23 des Smartphones 20 zu emittieren.

Somit kann für eine Photorefraktionsmessung eine gewünschte Exzentrizität 26 der Beleuchtung bereitgestellt werden, die dem Abstand eines effektiven Lichtaustrittsorts 210 des Beleuchtungslichts 24, der durch das Prisma 21 zu der Lichtquelle 22 versetzt ist, von der Kamera 23 (hier eingezeichnet zu einer Mittelachse entsprechend der optischen Achse 25 der Kamera) entspricht. Die Exzentrizität 26 ist hier wie eingangs erläutert von dem Lichtaustrittsort 210 zu der optischen Achse 25 der Kamera 23 gemessen. Ein vertikaler Versatz zwischen Lichtaustrittsort 210 und Kamera 23, d.h. ein Versatz in einer Richtung parallel zur optischen Achse 25 (hier eine Richtung senkrecht zu der Oberfläche des Smartphones 20) wird bei der Bestimmung der Exzentrizität vernachlässigt. Diesbezüglich ist es bevorzugt, das Prisma 21 so dünn (in einer Richtung parallel zu der optischen Achse 25) zu gestalten, dass dieser vertikale Versatz deutlich geringer als die Exzentrizität 26 ist. Die Exzentrizität 26 kann beispielsweise 5 mm betragen, was bei einem Abstand von 60 cm einen Messbereich von ca. -2,5 bis +2,5 Dioptrien ergibt.

Die Lichtquelle 22 ist bei dem dargestellten Ausführungsbeispiel eine Lichtquelle, welche auch im übrigen Betrieb des Smartphones 20 als Beleuchtung für Aufnahmen mit der Kamera 23 dient. Sie kann beispielsweise eine Blitzlichtquelle für Photoaufnahmen oder auch eine Dauerlichtquelle (beispielsweise auf Leuchtdioden basierend) für Videoaufnahmen mit dem Smartphone 20 sein.

Die Lichtquelle 22 kann auch eine Infrarotlichtquelle sein, die beispielsweise für einen Sensor des Smartphones 20 verwendet wird. Eine derartige Infrarotlichtquelle 22 kann auch weiter entfernt von der Kamera 23 angeordnet sein. In diesem Fall wird die Komponente 30 dann entsprechend dimensioniert, um eine Umlenkung zu einer gewünschten Exzentrizität, z. B. 5 mm wie oben beschrieben, zu erreichen. Der Wert von 5 mm ist hier nur als Beispiel zu verstehen, und je nach gewünschtem Arbeitsabstand und gewünschtem Messbereich können auch andere Werte, z. B. zwischen 5 mm und 20 mm, durch entsprechende Dimensionierung des Prismas 21 erreicht werden.

Das Prisma 21 kann dabei aus einem Glas oder einem Kunststoffmaterial gefertigt sein, was eine kostengünstige Fertigung ermöglicht. Ein derartiges Kunststoffprisma kann beispielsweise mit einem Spritzgussverfahren hergestellt werden. Somit kann mit dem Ausführungsbeispiel der Fig. 2 auf kostengünstige Weise bei einer gegebenen Entfernung zwischen der zu untersuchenden Person und dem Smartphone 20 ein jeweils gewünschter Messbereich für die exzentrische Photorefraktion bereitgestellt werden.

Das Prisma 21 ist also eingerichtet, für ein bestimmtes Modell des Smartphones 20, welches über einen bestimmten Abstand zwischen Lichtquelle 22 und Kamera 23 verfügt, eine gewünschte Exzentrizität bereitzustellen. Für verschiedene Typen von Smartphones 20 können dabei verschiedene Prismen angeboten werden.

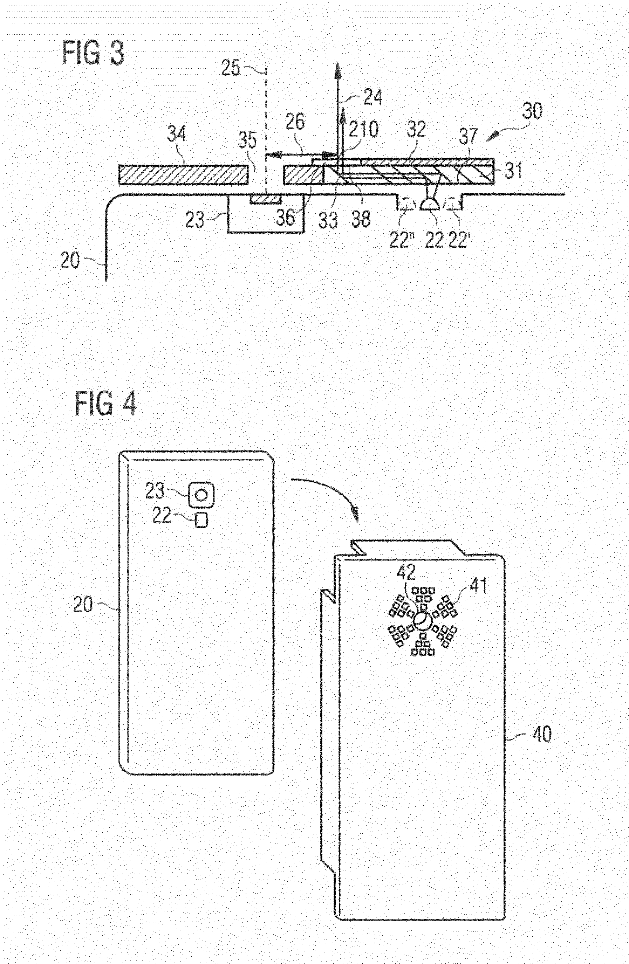

Die Fig. 3 zeigt eine Teilquerschnittsansicht eines Systems gemäß einem weiteren Ausführungsbeispiel. Das System umfasst das Smartphone 20 und eine erfindungsgemäße Komponente 30, welche wie die Komponente der Figur 2 an der Rückseite des Smartphones 20 angebracht ist.

Die in der Fig. 3 gezeigte erfindungsgemäße Komponente 30 ist derart eingerichtet, dass sie für verschiedene Positionen einer eingebauten Lichtquelle des Smartphones 20 eine gewünschte Exzentrizität 26 eines Lichtaustrittsorts 210 zu der optischen Achse 25 der Kamera 23 herstellen kann (die Erläuterungen zur Exzentrizität 26 als zwischen dem Lichtaustrittsort 210 und der optischen Achse 25 gemessen, welche unter Bezugnahme auf die Fig. 2 gemacht wurden, gelten auch für das Ausführungsbeispiel der Fig. 3). Die Komponente 30 umfasst ein Trägermaterial 34 mit einer Aussparung 35 für die Kamera 23 des Smartphones 20. In den Träger 34 ist eine Anordnung 31 erster reflektierender Flächen und eine zweite reflektierende Fläche eingearbeitet. Die Anordnung 31 ist bei dem dargestellten Beispiel ein Mikro-Prismen-Array, und die ersten reflektierenden Flächen sind Prismenflächen.

Licht von der Lichtquelle 22 gelangt durch eine Eintrittsfläche 37 zu der Anordnung 31 und wird von einer oder mehreren der ersten reflektierenden Flächen um etwa 90° in Richtung der zweiten reflektierenden Fläche 33 abgelenkt. An der zweiten reflektierenden Fläche 33 erfolgt wie in Fig. 3 dargestellt nochmals eine Ablenkung um etwa 90°, so dass der Lichtstrahl als das Beleuchtungslicht 24 mit der Exzentrizität 26 durch eine Austrittsfläche 38 ausgegeben wird. Es sind jedoch auch andere Geometrien mit anderen Winkeln möglich.

Die Exzentrizität 26 ist dabei im Wesentlichen, z. B. mit einer Toleranz von weniger als 1 mm oder weniger als 0,5 mm, unabhängig von der genauen Position der Lichtquelle 22. Zur Veranschaulichung sind in Fig. 3 andere mögliche Lichtquellen 22' und 22", welche zu der Lichtquelle 22 versetzt sind, in Fig. 3 dargestellt. Auch bei diesen Lichtquellen 22', 22" wird Licht von der Lichtquelle 22' oder 22" über die Anordnung 31 zu der zweiten reflektierenden Fläche 33 hin abgelenkt und von diesem wiederum abgelenkt, sodass sich unabhängig von der genauen Position der Lichtquelle stets im Wesentlichen die gleiche Exzentrizität 26 des Beleuchtungslichts 24 zu der optischen Achse 25 Kamera 23 ergibt.

Der Träger 34 ist bei dem dargestellten Beispiel als Folie ausgestaltet, welche auf einer Seite eine haftende Oberfläche aufweist, um so die optische Komponente 30 auf einfache Weise an dem Smartphone 20 befestigen zu können. Mit dem Bezugszeichen 32 ist eine lichtundurchlässige Abdeckung bezeichnet, welche verhindert, dass Licht, beispielsweise Streulicht, an anderen Stellen als der für das Beleuchtungslicht 24 vorgesehenen Stelle aus der Komponente 30 austritt.

Die Komponente 30 weist in dem in der Figur 3 dargestellten Ausführungsbeispiel einen optischen Filter 30 auf, nämlich einen chromatischen Bandpassfilter, um das Emissionsspektrum der Lichtquelle 22 auf ein gewünschtes Spektrum einzuschränken, beispielsweise ein Spektrum vorwiegend im roten und/oder infraroten Bereich wie eingangs erläutert. Der Filter 30 ist bei dem dargestellten Beispiel auf der Lichtaustrittsfläche 38 angeordnet. Ein derartiger Filter kann jedoch auch anderen Orten im Strahlengang, beispielsweise zwischen der Anordnung 31 und der zweiten Fläche 33, oder auch auf der Lichteintrittsfläche 37 angeordnet sein.

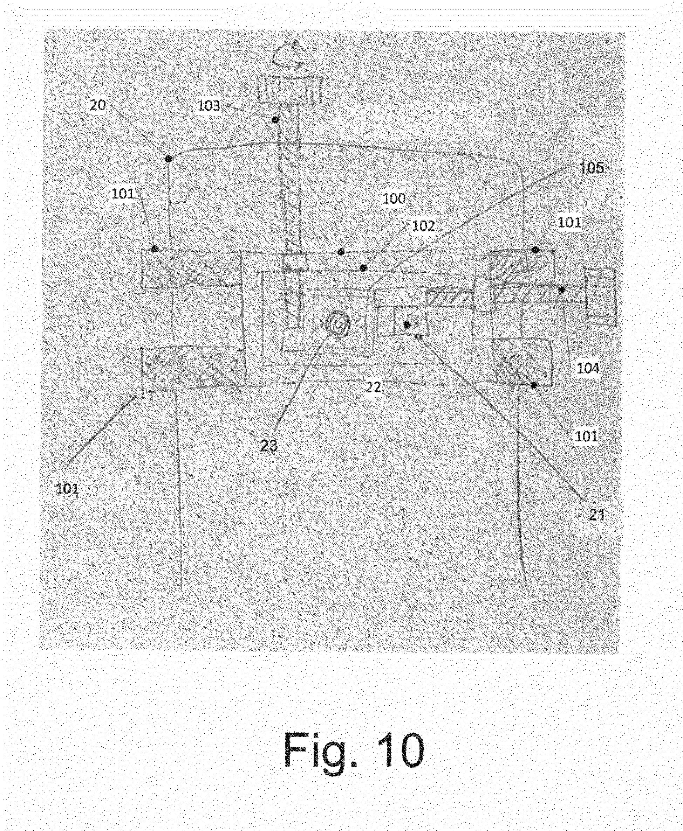

Die Fig. 10 zeigt ein System gemäß einem weiteren Ausführungsbeispiel, welches eine weitere Befestigungsmöglichkeit für das Prisma 21 an dem Smartphone 20 veranschaulicht. Das System der Fig. 10 weist dabei eine Platte 100 auf, welche mittels Klammern 101 an dem Smartphone 20 reversibel lösbar befestigt ist. Auf der Platte 100 ist eine weitere Platte 102 angeordnet, welche mittels Einstellschrauben 103, 104 relativ zu der Platte 100 bewegbar und positionierbar ist. Auf der weiteren Platte 102 ist das Prisma 21 befestigt.

Die Platten 100, 102 weisen eine Aussparung 105 für die Kamera 23 des Smartphones 20 auf. Des Weiteren weisen die Platten 100, 102 eine Aussparung für die integrierte Lichtquelle 22 auf.

Durch Bewegung der Platte 102 über die Einstellschrauben 103, 104 kann das Prisma 21 relativ zu der integrierten Lichtquelle 22 positioniert werden, sodass Licht von der Lichtquelle 22 auf die Fläche 27 des Prismas (siehe Fig. 2) trifft.

In den oben unter Bezugnahme auf die Figuren 2 und 3 diskutierten Ausführungsbeispielen wird Licht von einer Lichtquelle 22 des Smartphones 20 umgelenkt, um Beleuchtungslicht 24 mit einer gewünschten Exzentrizität 26 bereitzustellen. Bei den nachfolgend beschriebenen und in den Figuren 4 bis 6 gezeigten Ausführungsbeispielen weist die jeweilige Komponente selbst eine oder mehrere eigene Lichtquellen auf.

Das Ausführungsbeispiel der Fig. 4 zeigt eine Perspektivansicht eines Systems gemäß einem weiteren Ausführungsbeispiels mit dem Smartphone 20 und einer Komponente, welche in Form eines Hüllenelementes 40 ausgebildet ist.

Das Hüllenelement 40 umfasst eine Vielzahl von Lichtquellen 41. Die Vielzahl von Lichtquellen 41 sind in mehreren keilförmigen Anordnungen um eine Öffnung 42 in dem Hüllenelement 40, welches als Befestigungselement dient, angeordnet. Die Lichtquellen 41 sind beispielsweise Leuchtdioden (LEDs), organische Leuchtdioden (OLEDs) oder Quantenpunktlichtquellenelemente. Das Hüllenelement 40 ist an die gezeigte Art von Smartphone 20 derart angepasst, dass das Smartphone 20 in das Hüllenelement 40 eingesetzt werden kann und dabei die Kamera 23 des Smartphones 20 mit der Öffnung 42 zur Deckung kommt. Die Lichtquellen 41 sind dabei auf der Außenseite des Hüllenelements 40 angeordnet, d.h. auf der dem Smartphone 20 abgewandten Seite, wenn das Smartphone 20 in das Hüllenelement 40 eingesetzt ist. Auf diese Weise wird in dem dargestellten Beispiel die Lichtquelle 22 des Smartphones abgeschattet und gleichsam durch die Lichtquellen 41 ersetzt. Durch die Lichtquellen 41 kann eine Beleuchtung mit einer gewünschten Exzentrizität zu der Kamera 23 bereitgestellt werden. Durch die Verwendung mehrerer Lichtquellen wie in Fig. 4 ist die Exzentrizität zudem einstellbar. Durch die Anordnung in einer Keilform kann zudem einer Linearisierung des Rotreflexes in der Pupille erreicht werden, wie bereits weiter oben beschrieben. Zu bemerken ist, dass ein Hüllenelement wie das Hüllenelement 40 auch zum Befestigen der optischen Komponenten der Fig. 2 und 3 an einer mobilen Computervorrichtung wie einem Smartphone verwendet werden kann.

Die Lichtquellen 41 können im sichtbaren Bereich emittieren, können aber auch Infrarotlichtquellen mit Lichtemission unterhalb des sichtbaren Bereichs im Infraroten sein. Letzteres hat den Vorteil, dass die zu untersuchende Person weniger gestört wird und sich zudem die Pupille des zu untersuchenden Auges nicht oder weniger auf Grund der Beleuchtung durch Licht von den Lichtquellen 41 verengt. In diesem Fall ist in der Öffnung 42 ein Infrarotfilter (in Figur 4 nicht explizit gezeigt) bereitgestellt, der nur das Infrarotlicht passieren lässt.

Die Lichtquellenelemente 41 werden durch einen in dem Hüllenelement 40 angeordneten Akkumulator versorgt. Die Lichtquellenelemente 41 werden über das Smartphone 20 drahtgebunden oder drahtlos (beispielsweise über bluetooth oder NFC (Near Field Communication)) angesteuert. In anderen Implementierungen ist auch eine Ansteuerung mit einem in dem Hüllenelement 40 bereitgestellten Schalter oder anderem Bedienelement möglich.

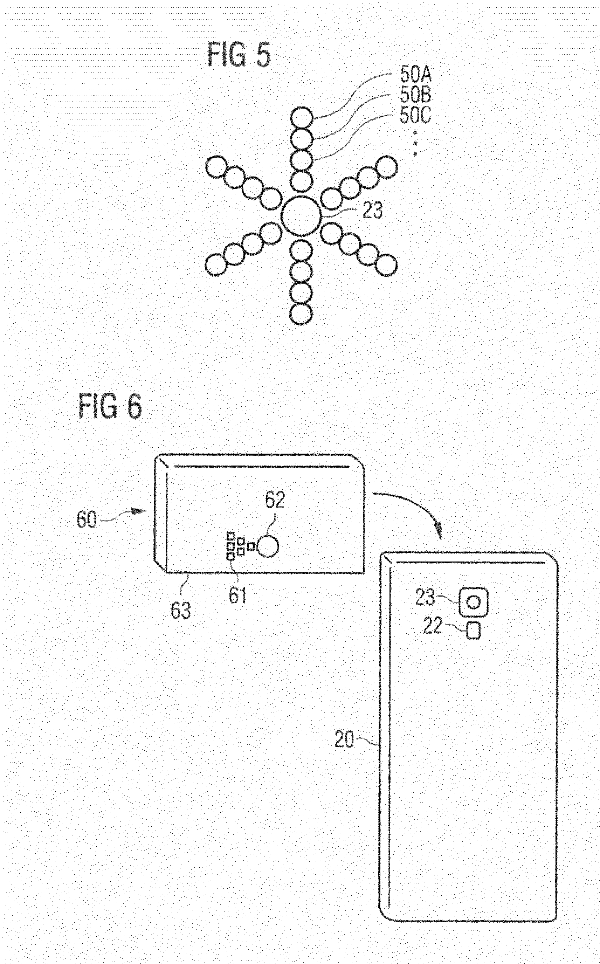

Es sind auch andere Anordnungen von Lichtquellenelementen oder andere Hüllenelemente als die in Fig. 4 dargestellten möglich. Die Fig. 5 zeigt ein System aus einem Smartphone, von dem in Fig. 5 lediglich die Kamera 23 dargestellt ist, und einer Komponente mit Optikelement, bei dem eine Vielzahl an Lichtquellenelementen 50A, 50B, 50C,..., bereitgestellt sind. Die Lichtquellenelemente 50A, 50B, 50C,... können beispielsweise in einem Hüllenelement wie dem Hüllenelement 40 der Fig. 4 angeordnet sein. Es sind bei dem Ausführungsbeispiel der Fig. 5 jeweils in einem bestimmten Winkel mehrere Lichtquellenelemente 50A, 50B, 50C,... in einer Reihe angeordnet. Durch wahlweises Einschalten der Lichtquellenelemente 50A, 50B, 50C,...kann eine gewünschte Exzentrizität realisiert werden. Bei 4 Lichtquellenelementen können diese beispielsweise in Abständen von 5 mm, 10 mm, 15 mm und 20 mm angeordnet sein. Dabei ist wie bereits erläutert jede Exzentrizität bei einer gegebenen Entfernung zwischen Smartphone und zu untersuchender Person einem definierten Messbereich, in dem eine exzentrische Photorefraktionsmessung möglich ist, zugeordnet. Mit dem obigen Zahlenbeispiel kann dann durch sukzessives Verwenden der 4 Lichtquellenelemente bei einem Abstand von 60 cm zwischen Smartphone und Auge insgesamt ein Messbereich zwischen etwa -9 und +9 Dioptrien abgedeckt werden. Die Lichtquellenelemente 50A, 50B, 50C,... können im sichtbaren und/oder im infraroten Bereich emittieren, wie bereits unter Bezugnahme auf Fig. 4 erläutert.

Die Fig. 6 zeigt ein System mit dem Smartphone 20 und einer Komponente 60. Die Komponente 60 umfasst ein Hüllenelement 63 und Lichtquellenelemente 61, welche benachbart zu einer Öffnung 62 in einem Hüllenelement 63 angebracht sind. Das Hüllenelement 63 ist in dem Ausführungsbeispiel der Fig. 6 kappenförmig und ist hinsichtlich Größe und Form an das Smartphone 20 angepasst, so dass es dem Smartphone 20 "aufgesetzt" werden kann. Dabei kommt ähnlich dem Ausführungsbeispiel der Fig. 4 die Öffnung 62 mit der Kamera 63 des Smartphones zur Deckung, und die interne Lichtquelle 22 des Smartphones 20 wird abgedeckt. Im Unterschied zu der Fig. 4 ist bei dem Ausführungsbeispiel der Fig. 6 nur ein einziger Keil an Lichtquellenelementen 61 vorhanden. Es können aber auch mehrere derartige Keile ähnlich der Fig. 4 oder eine Anordnung wie in Fig. 5 bereitgestellt sein. Dies zeigt insbesondere, dass verschiedene Ausgestaltungen von Hüllenelementen möglich sind, die mit verschiedenen Anordnungen von Lichtquellen kombiniert werden können. Auch die Lichtquellenelemente 61 können wie bereits unter Bezugnahme auf die Fig. 4 erläutert im Sichtbaren oder im Infraroten arbeiten, wobei im letzteren Fall in der Öffnung 62 ein entsprechender Infrarotfilter bereitgestellt sein kann, wie bereits für das Ausführungsbeispiel der Fig. 4 erläutert. Es ist zu bemerken, dass die Hüllenelemente 40 und 63 der Figuren 4 und 6 auch, statt Lichtquellenelemente aufzuweisen, mit dem Prisma 21 der Fig. 2 oder der Komponente 30 der Fig. 3 kombiniert sein können, um diese an dem Smartphone 20 anzubringen und zu positionieren. Auf diese Weise kann z. B. das Prisma 21 mittels des Hüllenelements relativ zu der internen Lichtquelle 22 des Smartphones 20 positioniert werden, um die Exzentrizität wie in Fig. 2 gezeigt anzupassen. Hierzu kann das Hüllenelement dann eine Öffnung an der Position der internen Lichtquelle 22 aufweisen, über der das Prisma 21 angeordnet ist.

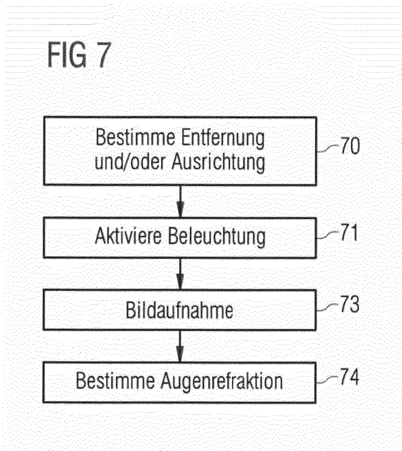

Im Folgenden wird nun erläutert, wie eine Augenglasbestimmung mit Hilfe eines der oben diskutierten Systeme aus mobiler Computervorrichtung und Komponente erfolgen kann. Hierzu zeigt Fig. 7 ein Flussdiagramm eines entsprechenden Verfahrens gemäß einem Ausführungsbeispiel. Das Verfahren kann dabei gesteuert durch die mobile Computervorrichtung durchgeführt werden, beispielsweise mittels eines in einem Speicher der mobilen Computervorrichtung (beispielsweise dem Speicher 17 der Fig. 1) abgelegten Computerprogramms (im Falle von Smartphones oder Tablets üblicherweise als App bezeichnet), welche auf einem Prozessor (wie dem Prozessor 15) ausgeführt wird und somit den Verfahrensablauf steuert.

Wie bereits oben erläutert, ist der Messbereich der exzentrischen Photorefraktionsmessung von der Exzentrizität der Beleuchtung, dem Pupillendurchmesser und von dem Abstand des Kopfes der zu untersuchenden Person zu der Kamera abhängig. Daher wird in Schritt 70 des Verfahrens der Fig. 7 zunächst die Entfernung zwischen der mobilen Computervorrichtung, beispielsweise dem Smartphone 20 der vorstehenden Ausführungsbeispiele, und dem Kopf der Person bestimmt (gemessen und/oder festgelegt), und/oder die Person wird angewiesen, die mobile Computervorrichtung in einer bestimmten Entfernung vom Kopf zu positionieren, beispielsweise zu halten. Eine derartige Entfernungsmessung bzw. die Festlegung der Entfernung zwischen mobiler Computervorrichtung und dem Kopf der Person kann beispielsweise auf Folgende Art und Weise erfolgen.

Es kann in Schritt 70 ein Objekt bekannter Größe in die Nähe des Gesichts der Person gebracht werden und ein entsprechendes Bild kann mit der Kamera aufgenommen werden. Dieses Bild kann das gleiche sein, welches für die für die Augenglasbestimmung verwendet wird. In diesem Fall erfolgt die Bestimmung des Abstands nicht in einem Schritt vorab, sondern zusammen mit der Aufnahme des Bildes. Aus der Größe des Objekts in dem Bild kann dann die Entfernung bestimmt werden. Eine derartige Entfernungsbestimmung ist jedoch auch vor der Aufnahme des Bildes, auf Basis dessen die Augenglasbestimmung erfolgt, möglich. Als Objekt bekannter Größe kann beispielsweise eine Kreditkarte oder ein anderer Gegenstand, der eine normierte oder bekannte Größe aufweist, dienen. Es kann z. B. auch ein Objekt mit definierten Abmessungen speziell für die Messung bereitgestellt werden. Ein derartiges Objekt bekannter Größe kann dann auch als Maßstab für weitere Zwecke dienen, beispielsweise zur Bestimmung von Abmessungen in dem aufgenommenen Bild wie beispielsweise der Pupillengröße.

Zur Bestimmung des Abstands in Schritt 70 kann auch eine Fokussiereinrichtung der Kamera der mobilen Computervorrichtung (Autofokus) verwendet werden. In Smartphones wie dem Smartphone 20 oder ähnlichen Computervorrichtungen verfügen die Kameras häufig über einen solchen Autofokus. Bei der Fokussierung mittels dieses Autofokuses wird der Abstand mit bestimmt, und diese Abstandsbestimmung kann dann auch für das erfindungsgemäße Verfahren verwendet werden. Auf diese Weise wird bereits vorhandene Hardware benutzt, um die Entfernung zu bestimmen.

Bei einer anderen Variante kann in Schritt 70 der oben erwähnte Autofokus der Kamera der mobilen Computervorrichtung auf eine fixe, für die Refraktionsmessung gewünschte Entfernung eingestellt werden. Die mobile Computervorrichtung kann dann eine positive Rückmeldung geben, wenn die Gesichtspartie, insbesondere die Augen, des Benutzers scharf abgebildet werden und sich der Benutzer somit in dem gewünschten Abstand befindet. Auf diese Weise kann der Abstand relativ einfach festgelegt werden.

Auch ein explizit vorhandener Abstandssensor (auch als Tiefensensor bezeichnet) oder ein Stereokamerasystem kann, soweit in der mobilen Computervorrichtung ohnehin vorhanden, in Schritt 70 zur Bestimmung des Abstands verwendet werden. Derartige Abstandssensoren können beispielsweise Infrarotsensoren, Laufzeitsensoren und dergleichen umfassen.

Bei einer anderen Variante kann zur Aufnahme des Gesichts des Benutzers ein Spiegel verwendet werden, d.h. der Benutzer wird nicht direkt aufgenommen, sondern über einen Spiegel. In einem derartigen Fall kann der Benutzer in Schritt 70 angewiesen werden, zur Bildaufnahme die mobile Computervorrichtung direkt neben ihr Gesicht zu halten, sodass die mobile Computervorrichtung im Spiegel zusammen mit dem Gesicht photographiert wird. In diesem Fall kann dann die mobile Computervorrichtung, beispielsweise das Smartphone, selbst als Größenmaßstab dienen (ähnlich wie bei dem obigen Fall, in dem ein bestimmter Gegenstand aufgenommen wird), da die Abmessungen der mobilen Computervorrichtung bekannt sind.

Bei wieder einem anderen Ausführungsbeispiel kann in Schritt 70 der zu untersuchende Benutzer oder eine Person, die die Untersuchung durchführt, aufgefordert werden, den Abstand manuell in der mobilen Computervorrichtung einzugeben. In diesem Fall kann also die Person (oder eine andere Person, die die Untersuchung durchführt, oder eine Hilfsperson) den Abstand manuell messen und dann eingeben.

In dem Fall, dass der Benutzer sich zur Untersuchung in der Art eines sogenannten Selfies selbst photographiert, kann auch die Armlänge des Benutzers in Schritt 70 abgeschätzt oder eingegeben werden (oder die Länge eines Hilfsmittels wie eines "Selfie Sticks", und basierend hierauf die Entfernung abgeschätzt werden.

Zusätzlich kann in Schritt 70 die Ausrichtung der mobilen Computervorrichtung, beispielsweise ein Winkel relativ zum Gesicht, bestimmt werden. Hierzu können interne Positions- und Lagesensoren der Computervorrichtung, wie sie beispielsweise in Smartphones üblicherweise vorhanden sind, verwendet werden.

In Schritt 71 wird dann die Beleuchtung unter Verwendung der an dem Gehäuse der mobilen Computervorrichtung angebrachten Komponente wie unter Bezugnahme auf die Figuren 1-6 beschrieben aktiviert. Bei den Systemen der Fig. 2 oder 3 wird hierzu die Lichtquelle 22 des Smartphones 20 aktiviert. Bei den Systemen der Fig. 4, 5 oder 6 werden Lichtquellenelemente 41, 50A-50C bzw. 61 der Komponente, insbesondere des Hüllenelements, aktiviert. Dies kann auch bereits vor dem Schritt 70 geschehen. Somit ist ersichtlich, dass die dargestellte Reihenfolge nicht als einschränkend auszulegen ist.

In Schritt 73 erfolgt dann mindestens eine Aufnahme eines Bildes der Person, insbesondere des Auges oder der Augen der Person, für die die Augenglasbestimmung durchgeführt werden soll. In Schritt 74 wird schließlich die Augenrefraktion mittels exzentrischer Photorefraktion auf Basis des in Schritt 73 aufgenommenen Bildes bestimmt.

Bei einem Ausführungsbeispiel wird hierzu zunächst die Pupille des zu untersuchenden Auges in dem aufgenommenen Bild detektiert und ein Pupillendurchmesser definiert, bei dem gerade keine Sättigung der Helligkeit in der Pupille mehr vorliegt. Dann wird das Helligkeitsprofil analysiert, wobei eine derartige Analyse für ein Farbbild (RGB-Bild - rot, grün, blau) oder auch nach Farbkanälen getrennt oder auch für einen Farbkanal über den gesamten Pupillendurchmesser erfolgen kann. Die Fehlsichtigkeit des Auges kann, im einfachsten Fall, aus der Steigung dieses Helligkeitsprofils ermittelt werden, beispielsweise auf Basis einer zuvor erzeugten Kalibrierung mit Augen bekannter Fehlsichtigkeit oder mit Optiken, die eine solche Fehlsichtigkeit simulieren.

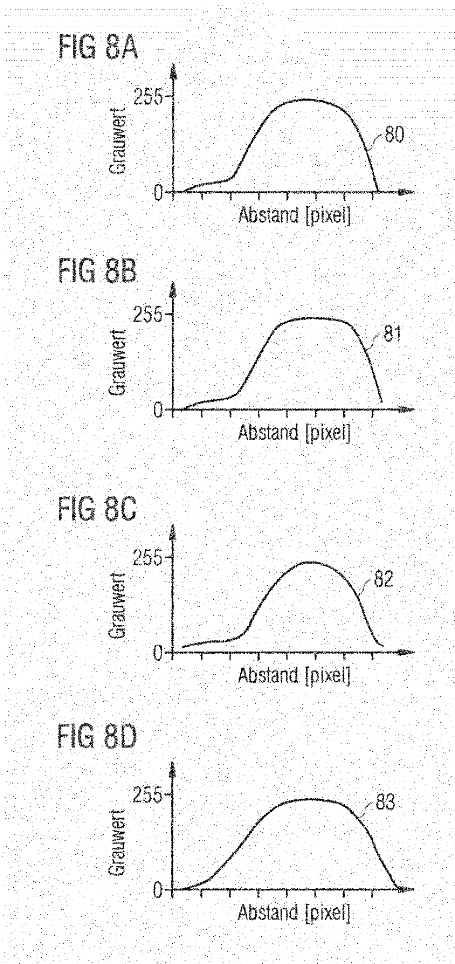

Als Beispiel zeigen die Figuren 8A-8D Beispiele für solche Helligkeitsprofile. Diese Beispiele dienen lediglich der Veranschaulichung, und unterscheiden sich beispielsweise je nach Fehlsichtigkeit des untersuchten Auges. Eine Kurve 80 in der Fig. 8A zeigt dabei eine Helligkeitsverteilung (Grauwert) in einem aufgenommen Farbbild eines Auges über den Durchmesser der Pupille (in Pixeln des Bildes). Eine Kurve 81 in Fig. 8B zeigt die Helligkeitsverteilung für den Rotanteil, eine Kurve 82 in Fig. 8C die Helligkeitsverteilung für den Grünanteil und eine Kurve 83 in Fig. 8D die Helligkeitsverteilung für den Blauanteil der Kurve 80 aus Fig. 8A. Die Helligkeit kann dabei wie in den Figuren 8A-8D dargestellt, entlang einer Linie über den gesamten Durchmesser der Pupille gemessen werden, kann jedoch beispielsweise auch innerhalb eines Teilbereichs des Auges, z. B. eines Rechtecks erfolgen. Über einen bekannten Konversionsfaktor bzw. bekannte Zusammenhänge wie bereits erläutert kann dann unmittelbar aus dem Anstieg der Helligkeit in der Pupille (d.h. beispielsweise dem Anstieg der Kurven 80 bis 83) auf die Refraktion des Auges in dem jeweiligen untersuchten Meridian geschlossen werden (d.h. ein Meridian, welcher durch eine Linie zwischen der Lichtquelle und der Kamera definiert wird). Für die Auswertung kann auch nur der Farbkanal mit der geringsten Streuung benutzt werden, was z. B. der blaue Farbkanal sein kann.

Die Schritte 71 und 73 können auch mehrmals durchgeführt werden, um mehrere Bilder mit verschiedenen Beleuchtungsrichtungen aufzunehmen, um eine Messung verschiedener Meridiane durchzuführen. Hierzu werden in Schritt 71 in dem System der Figur 4 nacheinander verschiedene Keile von Lichtquellen 41 aktiviert, um so nacheinander das zu untersuchende Auge aus verschiedenen Richtungen zu beleuchten, oder es werden in dem System der Fig. 5 Lichtquellen, die ausgehend von der Kamera 23 in verschiedenen Richtungen angeordnet sind, nacheinander aktiviert. Für jede Richtung wird dann in Schritt 73 ein Bild des Auges aufgenommen, womit ein Meridian, der durch die Position der aktivierten Lichtquelle und die Position der Kamera definiert ist, gemessen wird. In Schritt 74 erfolgt die Bestimmung der Augenrefraktion dann auf Basis der so aufgenommenen Bilder.

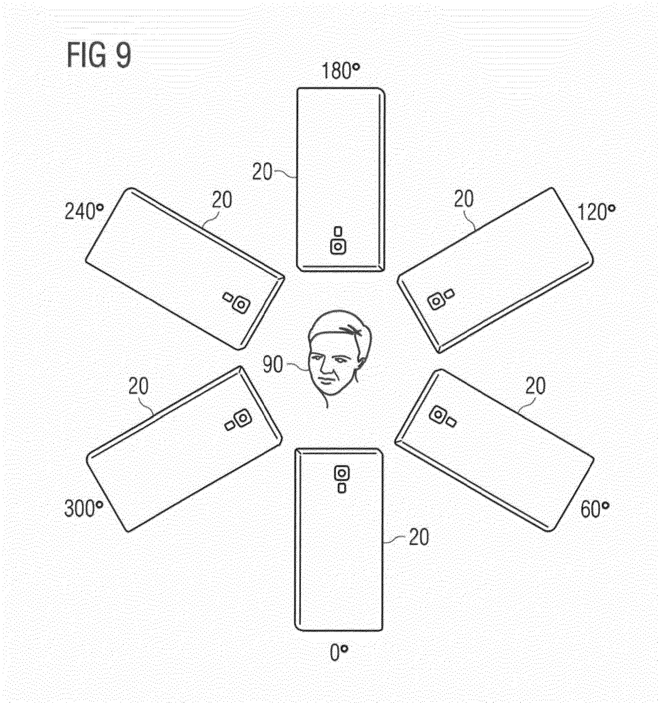

Für Systeme wie den Systemen der Fig. 1, 2 und 6, bei welchen eine oder mehrere Lichtquellen nur auf einer Seite der Kamera 23 des Smartphones 20 angeordnet sind, kann das Smartphone 20 zur Messung mehrerer Meridiane unter verschiedenen Winkeln zu dem Gesicht bei der Aufnahme positioniert werden, wie dies in Fig. 9 veranschaulicht ist. Die Fig. 9 zeigt das Smartphone 20 unter verschiedenen Winkelpositionen (z. B. 0°, 60°, 120°, 180°, 240°, 300°) zu dem Gesicht 90. Gegenüberliegende Winkelpositionen (z. B. 0° und 180°) messen dabei denselben Meridian und liefern daher keine zusätzlichen Informationen. Drei Winkel, die einen Abstand von 60° zueinander aufweisen (z. B. 0°, 60°, 180°), bieten dabei eine gute Abdeckung eines Halbkreises (180°), wobei der andere Halbkreis wie oben erläutert grundsätzlich die gleichen Informationen liefert. Dabei ist bei der Aufnahme dann die Kamera des Smartphones 20 dem Gesicht zugewandt und die optische Achse der Kamera kann für alle Aufnahmen im Wesentlichen die gleiche Position aufweisen. Auch wenn es in Fig. 9 nicht explizit dargestellt ist, ist das Smartphone 20 bei der Aufnahme dann mit einer entsprechenden optischen Komponente versehen (z. B. wie in Fig. 1-6 gezeigt). Bei einem bevorzugten Ausführungsbeispiel werden für eine Bestimmung von Sphäre, Zylinder und Achse eines zu vermessenden Auges die dargestellten Positionen 0°, 60° und 120° verwendet. Die anderen Positionen 180°, 240° und 300° können zusätzlich herangezogen werden, um die Genauigkeit der Messung (beispielsweise durch Mittelung) zu erhöhen. Der Winkel der mobilen Computervorrichtung kann dabei wie bei Schritt 70 der Fig. 7 erläutert durch interne Lagesensoren der mobilen Computervorrichtung bestimmt werden, wie sie beispielsweise bei Smartphones oder Tablet-Computern üblicherweise vorhanden sind.

Mit einer derartigen Messung mehrerer Meridiane können Aberrationen niedriger Ordnung, bei Augenuntersuchung üblicherweise als Sphäre, Zylinder und Achse bezeichnet, bestimmt werden. Hierzu werden wie erläutert mehrere Aufnahmen durchgeführt werden, wobei die Beleuchtung aus unterschiedlichen Richtungen erfolgt.

Dabei geht man üblicherweise von zwei Annahmen aus (1) die Achsen mit der größten und der geringsten Fehlsichtigkeit stehen senkrecht aufeinander und (2) die Fehlsichtigkeit ändert sich über die Meridiane nach dem Gaußschen Satz entsprechend einer Sinusquadrat-Funktion. Um diese Funktion an die Messwerte anpassen zu können, braucht man Messungen in mindestens drei Meridianen. Dies kann herkömmlicherweise zum Beispiel dadurch erreicht werden, indem ein "dreiarmiges" Skiaskop (d.h. Gerät zur Augenglasbestimmung) gebaut wird, bei dem die Kanten von einzelnen exzentrischen Photorefraktoren unter den Winkeln von 0°, 60° und 120° (oder jede andere Anordnung) angeordnet sind. Bei erfindungsgemäßen Ausführungsbeispielen werden Messungen verschiedener Meridiane wie oben erläutert durch Beleuchtung aus verschiedenen Richtungen durchgeführt.

Mittels derartiger Messungen in drei Meridianen werden dann Sphäre, Zylinder und Achse wie folgt berechnet, wobei in den folgenden Formeln R1 die bestimmte Refraktion bei einer Messung unter einer Beleuchtung aus 0°, R2 die Refraktion bei einer Messung bei Beleuchtung unter 60° und R3 bei einer Messung mit Beleuchtung unter 120° bezeichnet, wobei 0° einer Beleuchtung des Auges von unten entspricht. Für andere Richtungen können entsprechende Gleichungen aufgestellt werden:

$$Sph\ddot{a}re = A + \sqrt{B^2 + D^2}$$

$$Zylinder = A - \sqrt{B^2 + D^2}$$

$$Achse = 0{,}5 * \arctan(\frac{D}{B})$$

mit

$$A = \frac{R1 + R2 + R3}{3}$$

$$B = \frac{2 * R1 + R2 + R3}{3}$$

$$D = \frac{R2 - R3}{\sqrt{3}}$$

Genaueres zu derartigen Berechnungen findet sich in der Fachliteratur, z. B. Schaeffel F, Farkas L, Howland H. "Infrared photoretinoscope", Applied Optics 1987 oder Gekeler F, Schaeffel F, Howland HC, Wattam-Bell J. "Measurement of astigmatism by automated infrared photoretinoscopy". Measurement of astigmatism by automated infrared photoretinoscopy. Optom Vis Sci. 1997 Jul; 74(7):472-82.

Damit können mit den dargestellten Systemen aus einem mobilen Computervorrichtung und einer optischen Komponente Parameter wie Sphäre, Zylinder und Achse oder andere Informationen über die Refraktion mit einer kompakten Vorrichtung auf kostengünstige Weise bestimmt werden. So können Smartphones, Tablet-PCs und dergleichen mit den diskutierten optischen Komponenten und einer entsprechenden App ausgerüstet werden, um das Auge oder die Augen einer Person auf effektive Art und Weise vermessen zu können.

**Patentansprüche**

1. Verfahren zur Augenglasbestimmung mittels einer mobilen Computervorrichtung, umfassend:

   Bestimmen eines Abstands zwischen der mobilen Computervorrichtung und einem Kopf eines Benutzers,
   Aufnehmen eines Bildes eines Auges des Kopfes des Benutzers mit einer Kamera der mobilen Computervorrichtung, und
   Durchführen einer exzentrischen Photorefraktionsbestimmung auf Basis des aufgenommenen Bildes und des Abstands,
   **dadurch gekennzeichnet, dass** das Bestimmen des Abstands umfasst:

   - Bestimmen des Abstands auf Basis einer Autofokuseinstellung der Kamera (23) der mobilen Computervorrichtung, oder
   - Setzen eines Autofokus der Kamera (23) der mobilen Computervorrichtung auf einen vorgegebenen Abstandswert und Ausgeben einer Meldung, wenn ein Abstand des Kopfes dem vorgegebenen Abstandswert entspricht.

2. Computerprogramm für eine mobile Computervorrichtung mit einer Kamera (13), wobei das Computerprogramm, wenn es auf einem Prozessor (15) der mobilen Computervorrichtung (11) ausgeführt wird, bewirkt, dass das Verfahren gemäß Anspruch 1 durchgeführt wird.

3. Mobile Computervorrichtung (11; 20) mit einem Prozessor (15), einer Kamera (13) und einem auf einem Datenträger gespeicherten Computerprogramm nach Anspruch 2 zur Ausführung auf dem Prozessor.

**Claims**

1. Method for corrective lens determination by means of a mobile computer apparatus, comprising:

   determining a distance between the mobile computer apparatus and the head of a user,
   recording an image of an eye of the user's head using a camera of the mobile computer apparatus, and

performing an eccentric photorefraction determination based on the recorded image and the distance,
**characterized in that** the determination of the distance comprises

   - determining the distance based on an autofocus setting of the camera (23) of the mobile computer apparatus, or
   - setting an autofocus of the camera (23) of the mobile computer apparatus to a specified distance value and outputting a notification when a distance of the head corresponds to the specified distance value.

2. Computer program for a mobile computer apparatus having a camera (13), wherein the computer program, upon being executed on a processor (15) of the mobile computer apparatus (11), causes the method according to Claim 1 to be carried out.

3. Mobile computer apparatus (11; 20) having a processor (15), a camera (13) and a computer program, stored on a data carrier, according to Claim 2 for execution on the processor.

**Revendications**

1. Procédé de détermination de verre de lunette au moyen d'un dispositif informatique mobile, comprenant les étapes consistant à :

   déterminer une distance entre le dispositif informatique mobile et la tête d'un utilisateur,
   enregistrer une image d'un œil de la tête de l'utilisateur avec une caméra du dispositif informatique mobile, et
   effectuer une détermination par photoréfraction excentrique sur la base de l'image enregistrée et de la distance,
   **caractérisé en ce que** la détermination de la distance comprend :

   - la détermination de la distance sur la base d'un réglage d'autofocus de la caméra (23) du dispositif informatique mobile, ou
   - la définition d'un autofocus de la caméra (23) du dispositif informatique mobile sur une valeur de distance prédéfinie, et l'émission d'un message si une distance de la tête correspond à la valeur de distance prédéfinie.

2. Programme d'ordinateur pour un dispositif informatique mobile muni d'une caméra (13), dans lequel, lorsqu'il est exécuté sur un processeur (15) du dispositif informatique mobile (11), le programme d'ordinateur fait en sorte que le procédé selon la revendication 1 soit exécuté.

3. Dispositif informatique mobile (11 ; 20) avec un processeur (15), une caméra (13) et un programme d'ordinateur selon la revendication 2 stocké sur un support de données pour exécution sur le processeur.

## FIG 1

## FIG 2

FIG 3

FIG 4

FIG 5

50A
50B
50C
...

23

FIG 6

60

62

61

63

23

22

20

FIG 7

| | |
|---|---|
| Bestimme Entfernung und/oder Ausrichtung | 70 |
| Aktiviere Beleuchtung | 71 |
| Bildaufnahme | 73 |
| Bestimme Augenrefraktion | 74 |

FIG 8A

Grauwert

255

0

Abstand [pixel]

80

FIG 8B

Grauwert

255

0

Abstand [pixel]

81

FIG 8C

Grauwert

255

0

Abstand [pixel]

82

FIG 8D

Grauwert

255

0

Abstand [pixel]

83

FIG 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007031923 A1
- US 20150002817 A1
- DE 19719694 A1
- EP 1308128 A2
- US 2013235346 A
- DE 102015100147 A1
- DE 2232410 A


**In der Beschreibung aufgeführte Nicht-Patentliteratur**

- **HELMUT GOERSCH.** Wörterbuch der Optometrie. Verlag Bode GmbH & Co, 2001, 26
- **HELMUT GOERSCH.** Wörterbuch der Optometrie. Verlag Bode GmbH & Co, 2001, 239
- **W. WESEMANN ; H. I. WESEMANN.** Photorefraktion - Ein objektives Screening-Verfahren zur Refraktionsbestimmung. *DOZ - Deutsche Optiker Zeitung,* vol. 11 (92), 50-54
- **HOWLAND.** *statische Photoskiaskopie,* 1980
- **TYLER ; NOREIA.** *paraxiale Photorefraktion,* 1985
- **SCHAEFFEL et al.** *Photoretinoskopie,* 1987
- Photoscreening for Refractive Error and Strabismus With a Smartphone App. *ARVO (Association for Research in Vision and Ophthalmology) Konferenz 2014, Programmnr. 436, http://www.arvo.org/webs/am2014/abstract/ sessions/114.pdf*
- **SCHAEFFEL, F. ; FARKAS, L. ; HOWLAND, H.C.** Infrared photoretinoscope. *Appl. Opt.,* 1987, vol. 26, 1505-1509
- **SCHAEFFEL F ; FARKAS L ; HOWLAND H.** Infrared photoretinoscope. *Applied Optics,* 1987
- **GEKELER F ; SCHAEFFEL F ; HOWLAND HC ; WATTAM-BELL J.** Measurement of astigmatism by automated infrared photoretinoscopy. *Measurement of astigmatism by automated infrared photoretinoscopy. Optom Vis Sci.,* Juli 1997, vol. 74 (7), 472-82